# EUROPÄISCHE PATENTANMELDUNG

(11) **EP 1 417 925 A1**
(43) Veröffentlichungstag der Anmeldung: **12.05.2004**
(21) Anmeldenummer: 02405961.0
(22) Anmeldetag: 07.11.2002
(51) Int. Cl.: A61B 3/135, A61B 3/12, A61B 3/107, A61B 3/15

(54) **Vorrichtung zur Betrachtung eines Auges**

(71) Anmelder: HAAG-STREIT AG, CH-3098 Köniz (CH)
(72) Erfinder: Ulbers, Gerd, Dr., 3122 Kehrsatz (CH)
(74) Vertreter: Roshardt, Werner Alfred, Dipl.-Phys.

(57) **Zusammenfassung**

Das erfindungsgemässe Spaltlampengerät umfasst einen Tisch (1), einen mit einem Lenkhebel (8) verschiebbaren Kreuzschlitten (2), einen Kopfhalter (3), ein um eine Drehachse (9) drehbares Mikroskop (5) sowie auf einer Halterung (11) befestigte Beleuchtungsmittel (10). Die Halterung (11) umfasst eine ebenfalls um die Drehachse (9) drehbare Basis (11.1) und einen Träger (11.2), welcher mittels einer Schiene (13) relativ zur Basis (11.1) in Richtung des Auges (7) bzw. von diesem weg verschiebbar ist. Die Beleuchtungsmittel (10) sind auf dem Träger (11.2) befestigt und umfassen eine Linse (19), ein Prisma (18), eine Bildaufnahmevorrichtung (14) sowie eine Lichtquelle (17). Das Prisma (18) dient sowohl zur Umlenkung eines von der Lichtquelle (17) erzeugten Beleuchtungsstrahles auf das Auge (7), als auch zur Umlenkung eines aus dem Auge (7) austretenden Betrachtungsstrahles auf die Bildaufnahmevorrichtung (14), wobei diese zur Darstellung der so aufgenommenen Bilder an einen Computermonitor (16.2) angeschlossen ist. Werden die Beleuchtungsmittel (10) mit Hilfe der Schiene (13) direkt vor dem Auge (7) positioniert, kann mit diesem Spaltlampengerät auch der Augenhintergrund mit hoher Qualität untersucht werden.

## Beschreibung

### Technisches Gebiet

Die Erfindung betrifft eine Vorrichtung zur Betrachtung eines Auges eines Patienten, insbesondere ein Spaltlampengerät, umfassend Betrachtungsmittel zur Betrachtung eines vorderen Augenabschnittes des Auges sowie Beleuchtungsmittel zur Beleuchtung des vorderen Augenabschnittes des Auges.

### Stand der Technik

Für die Augenuntersuchung , -diagnostik und/oder -behandlung gibt es viele verschiedene Apparate und Vorrichtungen. Häufig kann dabei ein bestimmtes Gerät nur für eine Art von Untersuchung eingesetzt werden, da sich z. B. die Untersuchung der vorderen Augenabschnitte (Hornhaut etc.) grundlegend von der Untersuchung der hinteren Augenabschnitte (Fundus) unterscheidet. Dies unter anderem deshalb, weil bei der Untersuchung des Fundus dieser durch die Pupille hindurch beleuchtet werden muss, damit man überhaupt etwas erkennen kann. Bei der Untersuchung der vorderen Augenabschnitte hingegen können die zu untersuchenden Bereiche im Prinzip mit beliebigen Mitteln und von beliebigen Seiten beleuchtet werden. Selbst ohne eine künstliche Beleuchtung ist eine Betrachtung der vorderen Augenabschnitte möglich. Zur Typenvielfalt der Geräte trägt weiter bei, dass je nach zu untersuchenden Augenbereichen andere Abbildungseigenschaften möglich, erwünscht oder notwendig sind.

Für die Betrachtung der vorderen Augenabschnitte werden typischerweise sogenannte Spaltlampengeräte verwendet, wie sie beispielsweise in der WO 99/23937 A1 beschrieben sind. Solche Spaltlampengeräte umfassen in der Regel ein Mikroskop sowie eine Beleuchtungseinrichtung, welche gemeinsam verschiebbar auf einem Gerätefuss montiert und unabhängig voneinander um eine gemeinsame, vertikal liegende Drehachse drehbar gelagert sind. Das Licht der Beleuchtungseinrichtung wird über einen Umlenkspiegel auf das Auge gelenkt, welches mit dem Mikroskop betrachtet werden kann.

Für die Betrachtung der hinteren Augenabschnitte werden typischerweise ebenfalls spezielle Geräte, sogenannte Ophthalmoskope, verwendet. Diese gibt es in verschiedenen Ausführungsformen vom einfachen Augenspiegel bis hin zur hochkomplexen Funduskamera.

Beide Gerätetypen, Spaltlampengeräte und Ophthalmoskope haben jedoch ein gemeinsames Problem: Da sie zur Betrachtung der vorderen bzw. hinteren Augenabschnitte konzipiert sind, eignen sie sich jeweils gar nicht oder nur schlecht zur Betrachtung des jeweils anderen Augenabschnittes.

So ist es beispielsweise nicht möglich, mit einem Spaltlampengerät den Fundus zu betrachten. Hierfür müsste nämlich das Mikroskop unmittelbar vor dem Auge positioniert werden können, was aufgrund der vorgegebenen und nur in engen Grenzen veränderbaren Eigenschaften des Mikroskops und der Beleuchtung nicht möglich ist.

Eine Lösung dieses Problems besteht beispielsweise darin, zusätzliche optische Hilfsmittel zu verwenden, die in den Strahlengang eingeschaltet werden. So ist beispielsweise in der WO 01/87146 beschrieben, wie sich mit einem Spaltlampengerät und einer zwischen dem zu untersuchenden Auge und dem Mikroskop in den Strahlengang eingesetzten Vorsatzlinse der Fundus des Auges betrachten lässt. Allerdings lässt sich mit dieser Betrachtungstechnik nur eine relativ schlechte Abbildungsqualität erreichen, da bei dieser sowohl an der Hornhaut als auch an der Vorsatzlinse unerwünschte Lichtreflexionen entstehen, welche die Fundusbetrachtung erschweren. Zudem muss die Positionierung der Vorsatzlinse vor dem Auge entweder von Hand erfolgen, was selbst für einen Fachmann sehr schwierig ist, oder sie erfolgt mit Hilfe des Mikroskops, wobei die Vorsatzlinse danach zur Fundusbetrachtung, wie in der oben genannten Veröffentlichung beschrieben, vom Mikroskop abgekoppelt werden muss.

### Darstellung der Erfindung

Aufgabe der Erfindung ist es daher, eine Vorrichtung der eingangs genannten Art anzugeben, welche mit einem einzigen Gerät eine qualitativ hochstehende Betrachtung sowohl der vorderen als auch der hinteren Augenabschnitte erlaubt.

Die Lösung der Aufgabe ist durch die Merkmale des Anspruchs 1 definiert. Bei der erfindungsgemässen Vorrichtung handelt es sich um ein Gerät zur Betrachtung der vorderen Augenabschnitte, insbesondere um ein Spaltlampengerät, welches derart modifiziert ist, dass es auch für eine qualitativ hochstehende, reflexfreie Betrachtung des Augenhintergrundes (Fundus) eingesetzt werden kann. Die Vorrichtung umfasst Betrachtungsmittel zur Betrachtung eines vorderen Augenabschnittes, wobei die Betrachtungsmittel insbesondere ein auf einer Halterung befestigtes Mikroskop, beispielsweise ein Greenough-Mikroskop, umfassen. Weiter umfasst die Vorrichtung Beleuchtungsmittel zur Beleuchtung der vorderen Augenabschnitte, wobei die Beleuchtungsmittel eine Lichtquelle aufweisen, deren Licht direkt oder indirekt auf das Auge gerichtet wird.

Wie bei Spaltlampengeräten bekannt, sind die Betrachtungsmittel und die Beleuchtungsmittel mit Vorteil derart angeordnet, dass sie unabhängig voneinander um eine gemeinsame, vertikale Drehachse verschwenkt werden können. Wären sie starr montiert, könnte bei verschiedenen Augenuntersuchungen jeweils nur ein kleiner Bereich des Auges untersucht werden. Das erfindungsgemässe Spaltlampengerät umfasst insbesondere auch Fixierungsmittel. Diese dienen zur örtlichen Fixierung des Patientenkopfes und damit des zu untersuchenden Auges, um ungewollte Bewegungen des Patienten während einer Untersuchung möglichst zu vermeiden. Ohne einen derartigen Kopfhalter wäre es für den Patienten äusserst schwierig, minutenlang bewegungslos da zu sitzen.

Im Gegensatz zu den aus dem Stand der Technik bekannten Spaltlampengeräten ist die erfindungsgemässe Vorrichtung auch zur Betrachtung des Augenfundus geeignet, da einerseits die Beleuchtungsmittel derart ausgebildet sind, dass damit nicht nur die vorderen Augenabschnitte wie z. B. die Hornhaut, sondern auch ein hinterer Augenabschnitt wie z. B. der Fundus beleuchtbar ist. Andererseits weist die erfindungsgemässe Vorrichtung eine Bilderfassungsvorrichtung zur Erfassung von Bildern des Auges mit einer Bildaufnahmevorrichtung auf, welche derart ausgebildet und positionierbar ist, dass damit der hintere Augenabschnitt ophthalmoskopisch abgebildet werden kann. D. h. die Betrachtung bzw. Abbildung der hinteren Augenabschnitte erfolgt mit dieser zusätzlichen Bilderfassungsvorrichtung und nicht bzw. nicht ausschliesslich mit den Betrachtungsmitteln, mit welchen die vorderen Augenabschnitte betrachtet werden.

Für eine korrekte Abbildung der zu untersuchenden hinteren Augenbereiche umfasst die Vorrichtung zudem optische Abbildungsmittel wie beispielsweise eine oder mehrer Linsen, welche zwischen dem Auge und der Bildaufnahmevorrichtung positioniert ist bzw. sind.

Da zur Betrachtung des Fundus eine hierfür ausgebildete Bilderfassungsvorrichtung verwendet wird, entfällt die Notwendigkeit, die optischen Eigenschaften der zur Betrachtung der vorderen Augenabschnitte verwendeten Betrachtungsmittel zu verändern. Diese Bilderfassungsvorrichtung ist nun derart ausgebildet, dass sie bzw. bestimmte Teile davon, für eine ophthalmoskopische Betrachtung des Augenhintergrundes möglichst nahe vor dem Auge positioniert werden können. Dadurch wird es möglich, grosse Bereiche des Augenfundus mit einem Spaltlampengerät in bisher nicht dagewesener Qualität abzubilden und zu betrachten. Darüber hinaus ist es sogar möglich, mit dem zur Betrachtung der vorderen Augenabschnitte verwendeten Mikroskop gleichzeitig weitere, wenn auch kleinere Bereiche des Fundus zu betrachten.

Ebenso ist es möglich, die Bildaufnahmevorrichtung derart auszubilden, dass gleichzeitig mehrere Teilbilder des Augenfundus erfasst und derart kombiniert werden können, dass eine stereoskopische Betrachtung des Augenfundus möglich ist.

Wie weiter oben bereits erwähnt, umfassen die Beleuchtungsmittel eine Lichtquelle zur Erzeugung eines Beleuchtungsstrahles. Dieser wird direkt oder indirekt auf bzw. durch die Pupille hindurch in das Auge geworfen. Nun sind aber zur Beleuchtung der vorderen bzw. hinteren Augenabschnitte typischerweise unterschiedliche Beleuchtungen notwendig oder wünschenswert. Um die für die durchzuführende Untersuchung bestmögliche Beleuchtung zu erhalten, könnten die Beleuchtungsmittel daher beispielsweise modular ausgebildet sein, damit je nach gewünschter Beleuchtung beispielsweise eine andere Lichtquelle oder eine andere Optik eingesetzt werden könnte.

Es ist allerdings vorteilhafter, wenn die Beleuchtungsmittel derart ausgebildet sind, dass die Beleuchtungsart einfach von einer Vordergrundbeleuchtung auf eine Hintergrundbeleuchtung umgeschaltet werden kann. Dies sollte mit möglichst wenig Handgriffen, vorzugsweise per Knopfdruck oder durch Umlegen eines Hebels etc., durchgeführt werden können.

Die Bilderfassungsvorrichtung könnte beispielsweise derart ausgebildet sein, dass sie direkt in den Strahlengang eingefügt wird, d. h. dass die Bildaufnahmevorrichtung, beispielsweise eine Kamera, im richtigen Abstand direkt vor das Auge positioniert wird. Allerdings ist hierbei eine korrekte und genügende Beleuchtung des Augenfundus schwierig.

Vorzugsweise ist die Bilderfassungsvorrichtung daher derart ausgebildet, dass sie neben einer Bildaufnahmevorrichtung eine Umlenkvorrichtung umfasst. Um den Augenhintergrund ophthalmoskopisch abbilden zu können, muss hierbei lediglich die Umlenkvorrichtung im gewünschten Abstand nahe vor dem Auge positioniert werden. Die Umlenkvorrichtung kann genügend klein gefertigt sein, damit sie die Beleuchtung des Auges nicht wesentlich erschwert. Die Bildaufnahmevorrichtung muss dann nicht direkt vor dem Auge, sondern kann z. B. seitlich versetzt und wahlweise auch weiter entfernt angeordnet sein. Die Umlenkvorrichtung, welche vor dem Auge positioniert ist, lenkt einen aus dem Auge austretenden Betrachtungsstrahl, der auf die Umlenkvorrichtung trifft, auf die Bildaufnahmevorrichtung um.

Die Umlenkvorrichtung ist entsprechend derart ausgebildet, dass sie Licht in einem gewünschten Wellenlängenbereich mehr oder weniger stark in eine bestimmte Richtung reflektiert. Je nach gewünschter oder geforderter Reflexion bzw. anderer optischer Eigenschaften kann zur Umlenkung beispielsweise ein Spiegel oder ein Prisma verwendet werden. Die Verwendung eines Prismas zur Auskopplung bzw. Umlenkung des Betrachtungsstrahles wird jedoch bevorzugt, da ein Prisma typischerweise weniger Lichtverluste aufweist.

Selbstverständlich ist es möglich, die Bilderfassungsvorrichtung als separate Einheit auszubilden, welche bei Bedarf einfach in den Beobachtungs-Strahlengang eingebracht, beispielsweise eingeschwenkt werden kann. Dies erfordert jedoch viel Platz und erschwert die Handhabung des Spaltlampengerätes. Um Platz und Material zu sparen sowie eine möglichst einfache Handhabung zu gewährleisten, ist die erfindungsgemässe Vorrichtung mit Vorteil derart ausgebildet, dass die Beleuchtungsmittel und die Bilderfassungsvorrichtung eine Einheit bilden. D. h. die Bilderfassungsvorrichtung ist quasi in die bestehenden Beleuchtungsmittel, oder umgekehrt, integriert. Die Vorrichtung ist insbesondere derart ausgebildet, dass der von der Lichtquelle erzeugte Beleuchtungsstrahl von derselben Umlenkvorrichtung auf das Auge umgelenkt wird, welche auch zur Umlenkung eines aus dem Auge austretenden Betrachtungsstrahles auf die Bildaufnahmevorrichtung verwendet wird.

Die Lichtquelle zur Erzeugung des Beleuchtungsstrahles ist bei einem Spaltlampengerät in der Regel nicht direkt vor dem zu untersuchenden Auge positioniert, da sie in diesem Fall die Betrachtung des Auges mit den Betrachtungsmitteln stören würde. Der Beleuchtungsstrahl wird typischerweise ebenfalls über einen Spiegel auf das Auge geworfen. Auch hier kann anstatt des Spiegels beispielsweise ein Prisma verwendet werden. Die Beleuchtung kann nun derart ausgebildet sein, dass die Lichtquelle relativ zum Spiegel nicht verschiebbar ist. D. h. der Winkel, unter welchem der Beleuchtungsstrahl auf das Auge geworfen wird, wäre in diesem Fall unveränderbar. Die Beleuchtungsmittel sind vorzugsweise jedoch derart ausgebildet, dass dieser Winkel veränderbar ist, um unerwünschte und störende Lichtreflexe des Beleuchtungsstrahles möglichst zu vermeiden. Die Lichtquelle ist beispielsweise um eine horizontale Schwenkachse verschwenkbar angeordnet und kann so relativ zum Spiegel verschwenkt werden. Befindet sich die Lichtquelle beispielsweise im oberen Bereich des Spaltlampengerätes und erzeugt einen im Wesentlichen vertikal nach unten auf den Spiegel gerichteten Beleuchtungsstrahl, kann der Winkel, unter welchem der Beleuchtungsstrahl auf das Auge auftrifft, durch Verschwenken der Lichtquelle verändert werden.

Die Beleuchtungsmittel können in verschiedenster Art und Weise realisiert sein, wobei verschiedene Lichtquellen wie thermische Lichtquellen, Gasentladungslichtquellen oder Laserlichtquellen verwendet werden können. Es kann beispielsweise auch infrarotes Licht verwendet werden. Dadurch wird der Patient, dessen Auge untersucht werden soll, weniger geblendet und bei dunkler Umgebung adaptiert das Auge auf Dunkel, d. h. die Pupille des zu untersuchenden Auges ist weit geöffnet.

Das benötigte Licht kann natürlich auch an verschiedensten Orten erzeugt werden, sowohl direkt dort, von wo aus es auf das Auge gestrahlt wird, als auch an einem anderen Ort, wobei es in diesem Fall beispielsweise mittels Lichtleitern zum gewünschten Einsatzort geführt wird.

Bei einer bevorzugten Ausführungsform der Erfindung sind die Beleuchtungsmittel derart ausgebildet, dass der zur Beleuchtung des hinteren Augenabschnittes erzeugte Beleuchtungsstrahl eine Mehrzahl von auf das Auge gerichteten, ringförmig angeordneten Einzelstrahlen umfasst. Auch hier gilt, dass die Einzelstrahlen entweder direkt durch ringförmig angeordnete Lichtquellen, wie etwa Leuchtdioden, erzeugt und auf das Auge gerichtet werden oder dass die einzelnen Beleuchtungsstrahlen durch eine oder mehrere entfernt gelegene Lichtquellen erzeugt und z. B. mittels Lichtleitern, deren Enden ringförmig angeordnet sind, auf das Auge gerichtet werden. Selbstverständlich kann die ringförmige Beleuchtung auch erzeugt werden, indem das Licht einer Lichtquelle entsprechend geformt wird. Es könnte beispielsweise eine ringförmige Blende in den Beleuchtungsstrahlengang eingeführt werden, welche das Licht der Lichtquelle im Zentrum abblockt und nur in einem Ring um das Zentrum herum passieren lässt. Um die Untersuchung des Auges möglichst wenig zu beeinflussen, sind die Beleuchtungsstrahlen ringförmig um den Betrachtungsstrahl herum angeordnet.

Es gibt verschiedene Möglichkeiten zur Ausbildung des Spaltlampengerätes, damit die Bildaufnahmevorrichtung für eine ophthalmoskopische Abbildung des Augenfundus verwendet werden kann. Bilden die Bilderfassungsvorrichtung und die Beleuchtungsmittel, wie weiter oben erwähnt, beispielsweise eine Einheit, muss diese Einheit möglichst nahe vor dem Auge positioniert werden, damit eine ophthalmoskopische Abbildung des Augenfundus überhaupt möglich wird.

Bei herkömmlichen Spaltlampengeräten ist dies jedoch nicht möglich, denn sie weisen eine mechanische Drehachse bzw. Welle im Bereich des Patientenkopfes auf, um welche sowohl das Mikroskop, wie auch die Beleuchtungsmittel kreisförmig verschwenkbar sind. Genau diese mechanischen Achsen bzw. Wellen im Bereich direkt vor dem Patienten sind es jedoch, welche die Positionierung der Bilderfassungsvorrichtung direkt vor dem Auge verunmöglichen, weil sie bei deren Verschiebung in Richtung des Patienten an dessen Körper bzw. Kopf stossen würden, bevor sie nahe genug vor dem Auge positioniert sind, um den Fundus ophthalmoskopisch betrachten zu können. Eine Möglichkeit zur Lösung dieses Problems bestünde darin, die Halterung der Beleuchtung derart auszuführen, dass die mechanische Achse bzw. Welle nicht im Bereich direkt vor dem Patienten, sondern oberhalb oder unterhalb des Patientenkopfes liegt. Dies hat allerdings ein Spaltlampengerät zur Folge, welches in seinen Dimensionen erheblich grösser wird als herkömmliche Vorrichtungen. Dadurch ergeben sich unter Umständen auch unerwünschte Instabilitäten bei der Handhabung der Vorrichtung.

Eine weitere Möglichkeit besteht daher darin, die Drehachse, um welche die Beleuchtungsmittel und das Mikroskop verschwenkbar sind, nicht mittels mechanischer Achsen oder Wellen, sondern mittels schienenartiger Führungsmittel zu realisieren. D. h. die Beleuchtungsmittel sind beispielsweise via schienenartige Führungsmittel mit dem Vorrichtungsfuss verbunden. Die Beleuchtungsmittel können dann durch eine Verschwenkung entlang dieser schienenartigen Führungsmittel um die vertikale Drehachse gedreht werden. In gleicher Art und Weise könnte auch das Mikroskop via schienenartige Führungsmittel mit dem Vorrichtungsfuss verbunden sein. Zur Positionierung der Bilderfassungsvorrichtung müsste dann lediglich der Vorrichtungsfuss samt Mikroskop und Beleuchtung in Richtung des Patienten verschoben werden. Da keine mechanische Achse bzw. Welle mehr im Bereich vor dem Patienten vorhanden ist, kann der Vorrichtungsfuss ungehindert nahe genug vor dem zu untersuchenden Auge positioniert werden.

Bei einer bevorzugten Möglichkeit sind jedoch die Umlenkvorrichtung und die Bildaufnahmevorrichtung auf einer Halterung befestigt, welche eine Basis und einen Träger umfasst. Die beispielsweise länglich ausgebildete und horizontal liegende Basis ist, wie aus dem Stand der Technik bekannt, um eine vertikale, mechanische Drehachse bzw. Welle drehbar mit dem Spaltlampengerät verbunden. Im Gegensatz zu den bekannten Spaltlampengeräten, bei welchen die Beleuchtungsmittel nun starr mit der Basis verbunden sind, ist der längliche, vertikal angeordnete Träger hier über Führungsmittel mit der Basis verbunden.

Dadurch ist der Träger, an dessen oberen Ende die Umlenkvorrichtung angeordnet ist, relativ zur Basis verschiebbar.

Die Führungsmittel sind hierbei vorzugsweise schienenartig ausgebildet, sodass der Träger mit der Umlenkvorrichtung translatorisch entlang der Basis in Richtung des Auges bzw. von diesem weg verschiebbar ist. Hierbei wird die vertikale Drehachse, um welche die Umlenkvorrichtung verschwenkt werden kann, beibehalten. D. h. die Umlenkvorrichtung kann, selbst wenn sie extrem nahe vor dem Auge positioniert ist, um die vertikale Drehachse verdreht werden. Wird also der Vorrichtungsfuss des erfindungsgemässen Spaltlampengerätes derart positioniert, dass die vertikale Drehachse im Bereich des Auges verläuft, beispielsweise etwa in der Pupillenebene, können durch Verdrehen des Trägers mit der Umlenkvorrichtung durch die Pupille hindurch verschiedene Bereiche des Augenfundus untersucht werden.

Zur Aufnahme der Bilder kann im Grunde eine beliebige Bildaufnahmevorrichtung verwendet werden, wobei analoge Foto- bzw. Videokameras oder digitale Kameras mit einem CCD (charge coupled device) oder einem CMOS (Complementary Metall-Oxide Semiconductor) Bildchip bevorzugt eingesetzt werden.

Die aufgenommenen Bilder liegen dann in einem von der jeweiligen Kamera abhängigen Format vor. Wird z. B. eine Fotokamera mit einem lichtempfindlichen Filmstreifen verwendet, liegen die Abbildungen nach dem Entwickeln des Filmstreifens als Negativ bzw. Positiv vor. Bei herkömmlichen, analogen Videokameras mit Magnetbändern sind die Bildinformationen als analoges, magnetisches Muster auf den Bändern abgespeichert. Werden hingegen Digitalkameras wie z. B. eine digitale Foto- oder Videokamera verwendet, werden nicht Filmstreifen, sondern lichtempfindliche Chips mit einer Vielzahl von Pixeln belichtet. Bei solchen Chips können die erfassten Bilder direkt elektronisch weiter verarbeitet werden. Hierfür geeignet sind beispielsweise CCD Chips oder sogenannte CMOS Chips, wobei letztere den Vorteil haben, dass eine höherer Bildfrequenz erreicht und die elektronisch erfassten Bilder gleich mit einer auf demselben Chip untergebrachten CMOS Logik weiter verarbeitet werden können.

Die Bilder können beispielsweise in einem wählbaren, digitalen Bildformat auf einem beliebigen elektronischen Speichermedium abgespeichert werden. Sie können aber auch direkt auf einem Bildschirm, beispielsweise einem LCD-Bildschirm der Kamera selber oder auf einem Computermonitor als Einzelbilder oder auch als Bildfolgen mit wählbarer Bildfrequenz dargestellt werden. Die Art der Bilddarstellung ist beliebig und reicht vom normalen Fernseher mit entsprechendem Adapter über den bereits erwähnten Computerbildschirm bis hin zu Projektoren welche die elektronisch vorliegenden Bilder auf eine Wand oder Leinwand projizieren.

Entsprechend umfasst die Bilderfassungsvorrichtung vorzugsweise auch eine Bilddarstellungsvorrichtung zur Darstellung der Aufnahmen des Augenfundus. Die Darstellung kann sowohl in Echtzeit während der Augenuntersuchung erfolgen, als auch zu einem späteren Zeitpunkt, wobei die Aufnahmen dann einfach wieder aus dem verwendeten Speicher ausgelesen werden.

Die erwähnten optischen Abbildungsmittel zur Abbildung des Augenhintergrundes können sowohl augennah, d. h. in der Nähe des Auges, als auch kameranah, d. h. in der Nähe der Bildaufnahmevorrichtung vorgesehen sein, wobei die Positionierung nahe am Auge bevorzugt wird.

Die erfindungsgemässe Vorrichtung kann selbstverständlich auch weitere Komponenten aufweisen. So könnte die Bilderfassungsvorrichtung beispielsweise derart ausgebildet sein, dass bei der Fundusabbildung nur eine (vorgegebene) Vergrösserung möglich ist. Vorzugsweise ist die Bilderfassungsvorrichtung allerdings derart ausgebildet, dass sie beispielsweise mittels einer Wechseloptik, zwischen mehreren, d. h. wenigstens zwei Abbildungsvergrösserungen umschaltbar ist. D. h. mit einem oder mehreren zusätzlichen Linsensystemen, welche zwischen das Auge und die Bildaufnahmevorrichtung schaltbar sind, kann eine variable Vergrösserung erreicht werden.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: Eine schematische Darstellung einer erfindungsgemässen Vorrichtung zur Betrachtung der vorderen bzw. hinteren Augenabschnitte;
- Fig. 2: eine schematische Darstellung des Tisches mit der Fussplatte der Vorrichtung aus Fig. 1 von oben;
- Fig. 3: eine schematische Darstellung der Abbildungsverhältnisse mit der Vorrichtung aus Fig. 1;
- Fig. 4: eine schematische Darstellung einer Beleuchtungsvariante für die Vorrichtung aus Fig. 1;
- Fig. 5: eine schematische Darstellung einer weiteren Beleuchtungsvariante für die Vorrichtung aus Fig. 1;
- Fig. 6: eine schematische Darstellung eines Prismas für eine weitere Beleuchtungsvariante von der Seite;
- Fig. 7: das Prisma aus Fig. 6 von vorn, d. h. vom zu untersuchenden Auge aus gesehen;
- Fig. 8: eine schematische Darstellung eines anderen Ausführungsbeispiels der Erfindung;
- Fig. 9: eine schematische Darstellung des Tisches mit der Fussplatte der Vorrichtung aus Fig. 6 von oben;
- Fig. 10: eine schematische Darstellung einer als Spiegel ausgeführten Umlenkvorrichtung von der Seite in der Position zur Betrachtung des Augenhintergrundes;
- Fig. 11: die Umlenkvorrichtung aus Fig. 8 von vorn sowie
- Fig. 12: die Umlenkvorrichtung aus Fig. 8 von der Seite in der Position zur Betrachtung des Augenvordergrundes.

Grundsätzlich sind in den Figuren gleiche Teile mit gleichen Bezugszeichen versehen.

### Wege zur Ausführung der Erfindung

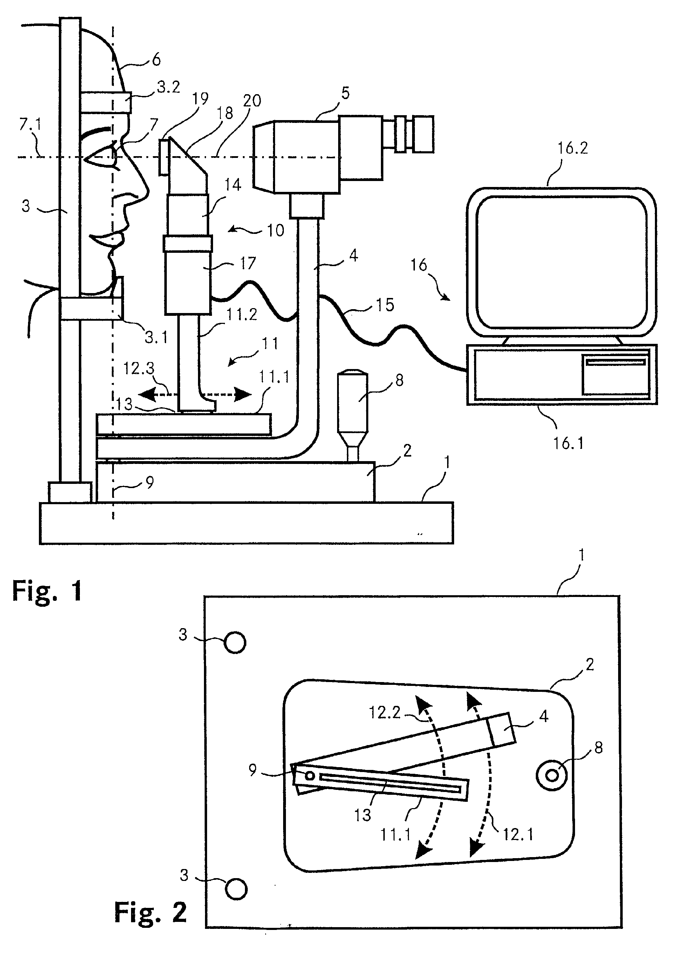

Die Figur 1 zeigt eine erfindungsgemässe Vorrichtung in einer schematischen Darstellung von der Seite, Die Vorrichtung umfasst einen Tisch 1, einen Kreuzschlitten 2, einen Kopfhalter 3 mit einer Kinnstütze 3.1 und einer Stirnstütze 3.2, ein auf einem Mikroskoparm 4 montiertes Mikroskop 5 sowie Beleuchtungsmittel 10, welche auf einer Halterung 11 befestigt sind. Der Mikroskoparm 4 und die Halterung 11 sind derart mit dem Kreuzschlitten 2 verbunden, dass sie kreisbogenförmig um eine gemeinsame, geometrische, vertikal liegende Drehachse 9 verschwenkt werden können. Am Ort der Drehachse 9 ist beispielsweise eine mechanische Achse vorgesehen, auf welche der Mikroskoparm 4 und die Halterung 11, welche mit entsprechenden Ausnehmungen versehen sind, aufgesteckt werden können. Im Kopfhalter 3 ist der Kopf 6 eines Patienten fixiert, dessen rechtes Auge 7 mit dem Mikroskop 5 untersucht werden soll.

Figur 2 zeigt den Tisch 1 und den Kreuzschlitten 2 von oben, wobei die vertikale Drehachse 9 bei dieser Ansicht lediglich punktförmig dargestellt ist. Die Drehbewegungen des Mikroskoparms 4 und der Basis 11.1 der Halterung 11 sind durch die gestrichelt eingezeichneten Pfeile 12.1 bzw. 12.2 dargestellt.

Der Kreuzschlitten 2 samt Beleuchtungsmittel 10 und Mikroskop 5 ist, wie aus dem Stand der Technik bekannt, mit Hilfe des Lenkhebels 8 auf dem Tisch 1 horizontal (beispielsweise durch Neigen des Lenkhebels 8) sowie vertikal (beispielsweise durch Drehen des Lenkhebels 8) verschiebbar. Mit dem Lenkhebel 8 kann der Kreuzschlitten 2 derart vor dem Kopf 6 des Patienten positioniert werden, dass auch das linke Auge (nicht sichtbar) mit dem Mikroskop 5 untersucht werden kann.

Die erfindungsgemässe Vorrichtung weist nun zumindest zwei wesentliche Unterschiede zu den aus dem Stand der Technik bekannten Spaltlampengeräten auf. Einerseits ist die Halterung 11 für die Beleuchtungsmittel 10 nicht wie beim Stand der Technik einstückig ausgebildet, sondern ist zweigeteilt, wobei der obere Teil der Halterung 11, der Träger 11.2, mit den Beleuchtungsmitteln 10 relativ zum unteren Teil der Halterung 11, der Basis 11.1, verschiebbar gelagert ist. Andererseits umfassen die Beleuchtungsmittel 10 nicht nur die Mittel zur Beleuchtung des Auges 7, sondern auch eine Bildaufnahmevorrichtung 14 zur Aufnahme von Bildern des Auges 7.

Weiter umfassen die Beleuchtungsmittel 10 eine Lichtquelle 17 zur Erzeugung der Beleuchtungsstrahlen sowie ein Prisma 18 zur Umlenkung der erzeugten Beleuchtungsstrahlen auf das Auge 7. Am Prisma 18 ist zudem eine Linse 19 befestigt, welche zur korrekten Abbildung des Augenhintergrundes benötigt wird. Um die Art der Beleuchtung an die durchzuführenden Untersuchungen anpassen zu können, verfügen die Beleuchtungsmittel 10 weiter über (nicht dargestellte) Mittel, beispielsweise zur Erzeugung verschiedener Querschnitte des Beleuchtungsstrahles wie Ringe oder Schlitze sowie beispielsweise Filter oder unterschiedliche Lichtquellen.

Ein von der Bildaufnahmevorrichtung 14 aufgenommenes Bild liegt beispielsweise elektronisch in Form von Pixelwerten vor. In dem in Figur 1 dargestellten Beispiel wird ein solches Bild über ein Kabel 15 an einen Computer 16 mit einem Rechner 16.1 und einem Monitor 16.2 übertragen und kann entweder direkt auf dem Monitor 16.2 dargestellt oder in einem Speicher des Rechners 16.1 abgespeichert werden. Die Darstellung eines Bildes oder einer Bildfolge kann entweder in Echtzeit oder sie kann anhand der abgespeicherten Bilder zu einem späteren Zeitpunkt erfolgen.

Anders als beim Stand der Technik ist die Halterung 11 der Beleuchtungsmittel nicht wie der Mikroskoparm 4 L-förmig ausgebildet, sondern umfasst wir bereits erwähnt eine Basis 11.1 und einen Träger 11.2. Die Basis 11.1 ist länglich ausgebildet und ist in einer horizontalen Ebene um die Drehachse 9 drehbar. Auf ihrer Oberseite weist die Basis 11.1 eine Schiene 13 auf. Der ebenfalls länglich ausgebildete, im Wesentlichen jedoch vertikal befestigte Träger 11.2 weist an seinem unteren Ende das zur Schiene 13 passende Gegenstück auf. Der Träger 11.2 kann beispielsweise mittels einer Rollen- oder Gleitlagerung entlang der Schiene 13 relativ zur Basis 11.1, d. h. in Richtung auf die Drehachse 9 zu oder von dieser weg in Richtung des Pfeiles 12.3, verschoben werden. Am oberen Ende des Trägers 11.2 sind die Beleuchtungsmittel 10 und sowie die Bildaufnahmevorrichtung 14 befestigt.

Ist der Kreuzschlitten 2 also vor dem zu untersuchenden Auge 7 positioniert, kann der Träger 11.2 zusammen mit den Beleuchtungsmitteln 10 und der Bildaufnahmevorrichtung 14 durch Verschieben auf der Basis 11.1 direkt vor das Auge 7 gebracht werden. Der Abstand der Linse 19 zum Auge 7 kann damit von wenigen Millimetern bis einigen Zentimetern beliebig eingestellt werden. Zur Betrachtung des Augenfundus beträgt der Abstand beispielsweise etwa 10 bis 15 mm. Ist der Kreuzschlitten 2 darüber hinaus derart vor dem Auge positioniert, dass die vertikale Drehachse 9 im Bereich des Auges 7, beispielsweise in deren Pupillenebene, liegt, können die Beleuchtungsmittel 10 und die Bildaufnahmevorrichtung 14 für jeden gewählten Abstand jeweils kreisförmig um das Auge 7 herum verschwenkt werden. Dadurch können durch die Pupille des Auges 7 hindurch jeweils unterschiedliche Bereiche des Augenhintergrundes ausgeleuchtet und untersucht werden.

Zur Untersuchung des Auges 7 kann die Vorrichtung derart vor dem Auge positioniert werden, dass die optischen Achsen 7.1 des Auges 7 und der Linse 19 im Wesentlichen übereinstimmen. Weiter ist die Vorrichtung derart ausgebildet, dass auch die optische Achse des Mikroskops 5 mit den optischen Achsen 7.1 der Linse 19 und des Auges 7 übereinstimmen. Dadurch ist es bei entsprechender Ausbildung des Prismas 18 aus (teilweise) transparentem Material auch möglich, das Auge 7 sowohl mit Hilfe der Bildaufnahmevorrichtung 14 und gleichzeitig mit Hilfe des Mikroskops 5 zu untersuchen.

Zur Ergänzung sei an dieser Stelle erwähnt, dass zusätzliche Mittel zur Feststellung der Halterung 11 sowie des Mikroskoparms 4 auf dem Kreuzschlitten 2 vorhanden sein können. Aus Gründen der Übersichtlichkeit wurden solche Mittel jedoch nicht dargestellt. Dasselbe gilt für die Mittel zur Begrenzung der Translationsbewegung des Trägers 11.2 auf der Basis 11.1.

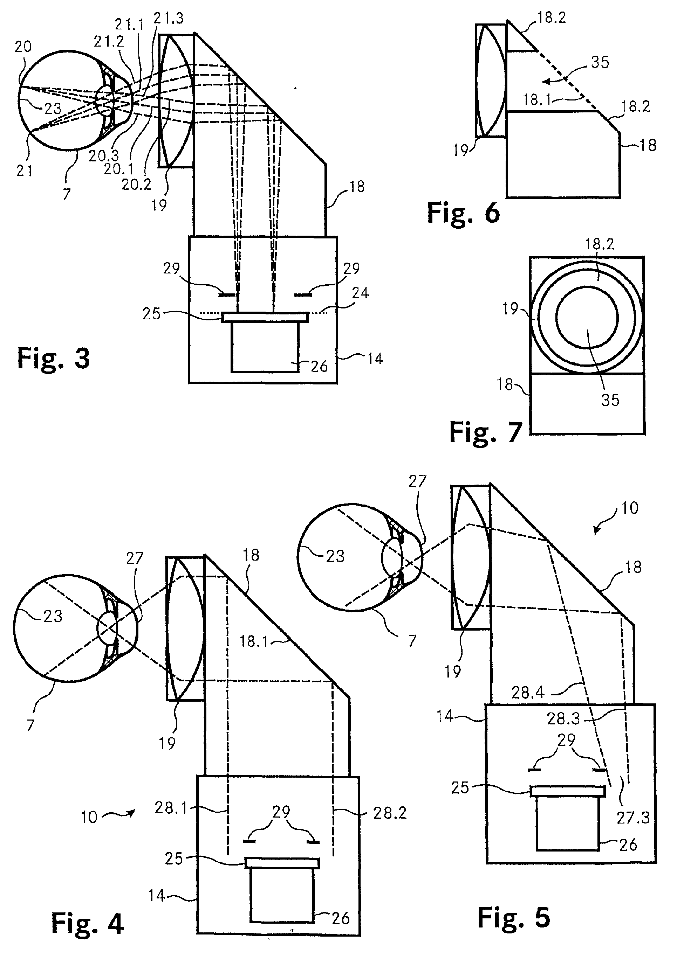

Die Figuren 3 bis 5 zeigen schematisch die Strahlengänge innerhalb der Beleuchtungsmittel 10.

Figur 3 zeigt insbesondere den Strahlengang für die Abbildung des Augenhintergrundes 23 des Auges 7. Stellvertretend für das gesamte Betrachtungsstrahlenbündel sind nur die Strahlen für zwei Funduspunkte 20 und 21 des Augenhintergrundes 23 dargestellt. Vom Funduspunkt 20 ausgehend sind drei Strahlen 20.1, 20.2, 20.3 dargestellt, wobei es sich beim Strahl 20.1 um den Mittelstrahl, d. h. den Strahl durch das Zentrum der Linse 19 handelt. Die beiden Strahlen 20.2 und 20.3 sind diejenigen Strahlen, die ausgehend vom Funduspunkt 20 oben bzw. unten gerade noch durch die Pupillenöffnung hindurch das Auge 7 verlassen können. Dasselbe gilt für den Funduspunkt 21 mit dem mittleren Strahl 21.1 und den seitlichen Strahlen 21.2 und 21.3. Sofern das Auge auf unendlich akkomodiert ist, verlaufen die Strahlen 20.1, 20.2, 20.3 des Funduspunktes 20 ausserhalb des Auges 7 parallel. Dasselbe gilt auch für die vom Funduspunkt 21 ausgehenden Strahlen.

Die Linse 19 fokussiert die Strahlen eines Funduspunktes auf eine virtuelle Bildebene. Durch Einfügen des Prismas 18 in den Strahlengang werden die Strahlen jedoch derart umgelenkt, dass ein Abbild des Augenhintergrundes 23 in einer Bildebene 24 entsteht. In dieser Bildebene 24 ist nun ein CCD-Chip 25 der Bildaufnahmevorrichtung 14 angeordnet, sodass damit das Abbild des Augenhintergrundes 23 aufgenommen werden kann. Das Auslesen und Weiterverarbeiten des Bildes erfolgt beispielsweise mittels einer Elektronik 26, welche ebenfalls in der Bildaufnahmevorrichtung 14 vorhanden ist. Das in Figur 1 dargestellte Kabel 15 zur Übertragung der Bilddaten an einen Computer wäre beispielsweise an diese Elektronik 26 angeschlossen (nicht dargestellt).

Um unerwünschte Fremdlichteinflüsse wie z. B. auf den CCD-Chip 25 fallende Lichtreflexe möglichst zu minimieren, ist eine entsprechend ausgebildete Ringblende 29 in den Strahlengang eingefügt. Derartige Lichtreflexe entstehen beispielsweise an den inneren oder äusseren Grenzflächen des Prismas 18, der Linse 19 oder des Auges 7.

Der Abstand des CCD-Chips 25 vom Auge 7 bzw. der Linse 19 kann durch Verschieben des Trägers 11.2 eingestellt werden. Für eine noch präzisere Positionierung könnte die Vorrichtung zusätzliche, nicht dargestellte Mittel zur Feinjustierung des Abstandes CCD-Chip 25 - Auge 7 umfassen. Diese Feinjustierung kann entweder durch eine horizontale Verschiebung des Trägers 11.2 relativ zur Basis 1.1 oder durch eine entsprechende vertikale Verschiebung des CCD-Chips 25 erreicht werden.

Figur 4 zeigt eine Möglichkeit zur Ausleuchtung des Augenhintergrundes 23 mit einer ringförmigen Beleuchtung. Die Lichtquelle selber, welche im unteren Teil der Beleuchtungsmittel 10 positioniert ist, ist nicht dargestellt. Die Lichtquelle erzeugt jedoch eine Mehrzahl von ringförmig um den CCD-Chip 25 angeordneten Lichtbündeln. Die Erzeugung dieser Lichtbündel erfolgt beispielsweise mit entsprechenden Blenden oder das durch die Lichtquelle erzeugte Licht wird mittels Linsen und Spiegeln oder anderen optischen Bauteilen in der gewünschten Art und Weise geformt, sodass die benötigte Ausleuchtung des Augenhintergrundes erreicht wird.

Für zwei dieser Lichtbündel ist jeweils ein Strahl 28.1, 28.2 eingezeichnet. Die Strahlen 28.1, 28.2 werden vom Prisma 18 umgelenkt und durch die Linse 19 hindurch auf das Auge 7 geworfen, wobei die Beleuchtungsmittel 10 derart ausgebildet sind, dass die ringförmig angeordneten Strahlen 28.1, 28.2 auf bzw. ein wenig vor oder hinter der Hornhaut 27 des Auges 7 ringförmig abgebildet werden. Dadurch entstehen keine störenden Reflexe im Bereich der Hornhaut 27 und es gelangt ein möglichst grosser Teil der Lichtbündel durch die Pupillenöffnung des Auges 7 hindurch auf den Augenhintergrund 23.

In Figur 5 ist eine weitere Möglichkeit zur Beleuchtung des Augenhintergrundes 23 dargestellt. Die Beleuchtung erfolgt hier nicht ringförmig, sondern umfasst lediglich ein von der (nicht dargestellten) Lichtquelle ausgehendes, seitlich einfallendes Strahlenbündel, von welchem die begrenzenden Strahlen 28.3, 28.4 dargestellt sind. Diese werden vom Prisma 18 via Linse 19 und Pupille wiederum auf den Augenhintergrund 23 des Auges 7 umgelenkt. Auch hier gilt, dass die Strahlen 28.3, 28.4 auf bzw. ein wenig vor oder hinter der Hornhaut 27 des Auges 7 abgebildet werden. Durch eine seitliche Einkopplung des Beleuchtungsstrahles bei anpassbarem Winkel können sich mit der Blende 29 zudem unerwünschte und die Augenuntersuchung störende Reflexe vermeiden lassen.

Die Lichtquelle, welche einen derart seitlich einfallenden Beleuchtungsstrahl erzeugt, muss hierbei nicht unbedingt in die Beleuchtungsmittel 10 integriert sein. Sie kann auch ausserhalb der Beleuchtungsmittel 10 vorgesehen sein, wobei der erzeugte Beleuchtungsstrahl entweder direkt von der Seite auf das Auge 7 gerichtet, oder aber seitlich in ähnlicher Weise wie in Fig. 5 dargestellt, in das Prisma 18 gerichtet und von diesem auf das Auge 7 umgelenkt wird.

Die Beleuchtung kann auch in an sich bekannter Art und Weise realisiert sein. Die Lichtquelle befindet sich hierbei im oberen Bereich der Vorrichtung und erzeugt ein nach unten gerichtetes Lichtbündel. Die Lichtquelle sitzt auf einer Halterung, welche selber derart an einem Träger befestigt ist, dass sich die Halterung samt Lichtquelle um eine horizontale Drehachse verschwenken lässt. Am oberen Ende des Trägers befindet sich ein Spiegel, welcher das von der Lichtquelle erzeugte und nach unten gerichtete Lichtbündel auf das Auge umlenkt. D. h. das Lichtbündel trifft ungefähr in einem Winkel von 45 Grad auf den Spiegel und wird von diesem mehr oder weniger horizontal auf das Auge umgelenkt. Durch leichtes Verschwenken der Halterung mit der Lichtquelle um die horizontale Drehachse lässt sich der Winkel, unter welchem das Lichtbündel auf das Auge trifft, verändern. Auch bei einem derart ausgebildeten Spaltlampengerät liesse sich der Träger in der erfindungsgemässen Art und Weise horizontal verschiebbar anordnen, sodass die Halterung samt Lichtquelle nahe vor dem Auge positioniert werden könnte. Weiter könnte auch hierbei eine Kamera in die Beleuchtung integriert werden, um das aus dem Auge austretende Licht über den am oberen Ende des Trägers befestigten Spiegel auf die Kamera umzulenken.

Die Figuren 6 und 7 zeigen ein weiteres Beispiel, wie eine ringförmige Beleuchtung des Fundus erreicht werden kann. Im Gegensatz zum Prisma 18 aus Fig. 4, bei welchem die gesamte Umlenkfläche 18.1 Licht reflektiert, reflektiert das in Fig. 6 im Querschnitt und in Fig. 7 von vorn dargestellte Prisma 18 Licht nur in einem ringförmigen Bereich 18.2 der gestrichelt dargestellten Umlenkfläche 18.1. Bei einer Beleuchtung des Prismas 18 von unten entsteht damit ebenfalls eine ringförmige Beleuchtung des Augenhintergrundes 23.

Der ringförmig reflektierende Bereich 18.2 könnte dadurch erzeugt werden, dass die Umlenkfläche 18.1 nur in einem entsprechenden Bereich reflektierend ausgebildet, beispielsweise verspiegelt, ist. Es ist aber, wie dargestellt, auch möglich, den nicht reflektierenden Bereich des Prismas einfach zu entfernen. Dadurch entsteht im Prisma 18 vom Auge 7 aus gesehen quasi direkt hinter der Linse 19 ein horizontal liegendes Loch 35. Durch dieses Loch 35 können Strahlen, welche aus dem Auge 7 austreten und durch den zentralen Bereich der Linse 19 verlaufen, ungehindert durch das Prisma 18 hindurch zu einem dahinterliegenden (nicht dargestellten) Mikroskop oder einer weiteren Kamera gelangen, mit welchem bzw. welcher auf diese Weise ebenfalls eine Fundusbetrachtung möglich ist. Das Prisma 18 mit dem Loch 35 wirkt hierbei gleichzeitig als Blende, welche unerwünschte Reflexe aus dem Bereich des Auges 7 oder der Linse 19 abblockt.

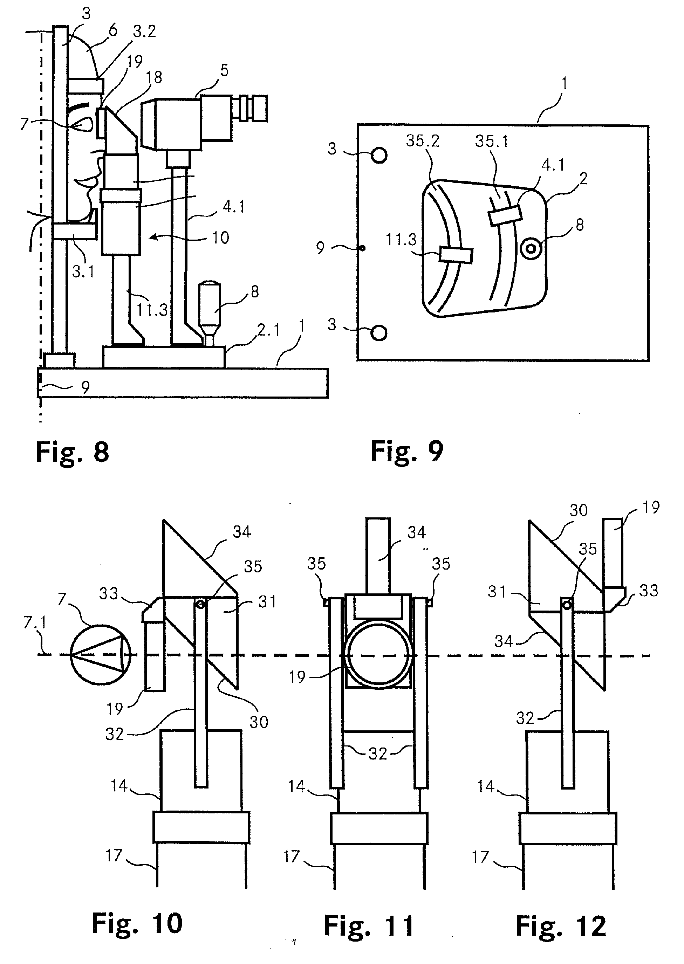

Fig. 8 zeigt eine weiteres Beispiel zur Ausführung der Erfindung. Das dargestellte Spaltlampengerät ist bis auf die Halterungen für das Mikroskop 5 und die Beleuchtungsmittel 10 gleich ausgebildet wie das in Figur 1 dargestellte Gerät. In Figur 9 ist wiederum der Tisch 1 mit dem Kreuzschlitten 2.1 von oben dargestellt.

Das Mikroskop 5 ist hier nicht über einen L-förmigen Arm, sondern über einen vertikal angeordneten länglichen Mikroskoparm 4.1 mit dem Kreuzschlitten 2.1 verbunden. Der Mikroskoparm 4.1 wiederum ist über eine Schiene 35.1, welche kreisförmig ausgebildet ist und sich auf der Oberseite des Kreuzschlitten 2.1 befindet, mit dem Kreuzschlitten 2.1 verbunden.

In ähnlicher Weise ist auch die Halterung 11 der Beleuchtungsmittel 10 ausgebildet. Der Träger 11.3, auf welchem das Prisma 18, die Bildaufnahmevorrichtung 14 und die Lichtquelle 17 befestigt sind, ist über eine Schiene 35.2 mit dem Kreuzschlitten 2.1 verbunden. Auch diese Schiene 35.2 ist kreisförmig ausgebildet und befindet sich auf der Oberseite des Kreuzschlittens 2.1. Sowohl der Mikroskoparm 4.1 als auch der Träger 11.3 können mittels einer Rollen- oder Gleitlagerung kreisförmig entlang den Schienen 35.1 bzw. 35.2 verschwenkt werden. Beide beschreiben hierbei eine kreisförmige Bewegung um eine virtuelle Drehachse 9.1.

Zur Untersuchung des Augenvordergrundes (nicht dargestellt) wird der Kreuzschlitten 2.1 samt Mikroskop 5 und Beleuchtungsmittel 10 mit Hilfe des Lenkhebels 8 in dem erforderlichen Abstand vor dem Auge positioniert. Die Radien der Schienen 35.1, 35.2 sind hierbei derart gewählt, dass die virtuelle Drehachse 9.1 bei dieser Position des Kreuzschlittens 2.1 im Bereich des Auges 7 verläuft. Durch Verschwenken des Mikroskops 5 bzw. der Beleuchtungsmittel 10 kann somit die Oberfläche des Auges 7 aus verschiedenen Winkeln betrachtet bzw. beleuchtet werden.

Soll nun, wie in Fig. 8 dargestellt, der Augenhintergrund des Auges 7 untersucht werden, kann der Kreuzschlitten 2.1 samt Mikroskop 5 und Beleuchtungsmitteln 10 mit Hilfe des Lenkhebels 8 näher zum Auge 7 hin verschoben werden, bis das Prisma 18 mit der Linse 19 schliesslich direkt vor dem Auge 7 positioniert ist. Dies ist bei herkömmlichen Spaltlampengeräten nicht möglich, da die Drehachse, um welche das Mikroskop 5 verdreht werden kann, als mechanische Achse bzw. Welle realisiert ist und diese beim Verschieben des Kreuzschlittens hin zum Auge 7 mit dem Körper des Patienten kollidieren würde, bevor das Prisma 18 genügend nahe am Auge 7 ist. Bei der erfindungsgemässen Vorrichtung wird eine solche Kollision vermieden, da die Drehachse 9.1 durch die kreisförmigen Schienen 35.1, 35.2 realisiert und damit nur virtuell, jedoch nicht real vorhanden ist.

In den Figuren 10 bis 12 ist eine weitere Variante der Ausbildung der Umlenkvorrichtung dargestellt, bei welcher die Umlenkvorrichtung als Spiegel 30 realisiert ist. Der Spiegel ist an einer Halterung 31 befestigt, welche über ein Gestänge 32 mit der Bildaufnahmevorrichtung 14 verbunden ist. Das Gestänge 32 hält die Halterung in einem bestimmten Abstand über der Bildaufnahmevorrichtung 14. Das aus der Bildaufnahmevorrichtung 14 austretende Licht zur Beleuchtung des Fundus des Auges 7 wird vom Spiegel 30 durch die Linse 19 hindurch auf das Auge 7 umgelenkt. Umgekehrt passiert das aus dem Auge 7 austretende Licht die Linse 19 und wird vom Spiegel 30 auf die Bildaufnahmevorrichtung 14 umgelenkt. Die Linse 19 wird hierbei von einem Linsenhalter 33 vor dem Spiegel 30 gehalten, wobei der Linsenhalter 33 selber an der Halterung 31 befestigt ist. Figur 10 zeigt die Umlenkvorrichtung von der Seite, Figur 11 von vorn, d. h. vom Auge 7 aus gesehen.

Um eine möglichst gute Abbildung des Fundus zu erreichen, muss der Spiegel 30 hierbei zumindest so breit wie der Durchmesser der Linse 19 sein. Dies ist am besten aus Fig. 11 ersichtlich.

Ein solch breiter Spiegel 30 würde allerdings stören, wenn der Augenvordergrund mit dem Mikroskop 5 in herkömmlicher Manier betrachtet werden soll. Er würde nämlich die Sicht vom Mikroskop 5 auf das Auge 7 versperren. Um dieses Spaltlampengerät auch für die Untersuchung des Augenvordergrundes verwenden zu können, muss daher der breite Spiegel 30 durch einen schmaleren Spiegel ersetzt werden können.

Die in den Figuren 10 bis 12 dargestellte Umlenkvorrichtung ermöglicht dies auf einfachste Art und Weise. Auf der Oberseite der Halterung 31 ist nämlich ein schmaler Spiegel 34 befestigt, wobei die Halterung 31 derart mit dem Gestänge 32 verbunden ist, dass sie um die horizontale Drehachse 35, welche im oberen Bereich des Gestänges 32 entsprechend gelagert ist, gedreht werden kann. D. h. je nach gewünschter Untersuchung kann durch einfaches Drehen der Halterung 31 entweder der breite Spiegel 30 zusammen mit der Linse 19 oder nur der schmale Spiegel 34 in den Strahlengang eingebracht werden. Zur genauen Positionierung kann die Umlenkvorrichtung auch Mittel aufweisen, um die Halterung 31 in den gewünschten Positionen einrasten zu lassen.

Figur 12 zeigt die Umlenkvorrichtung in der Position, in welcher sich der schmale Spiegel 34 im Beleuchtungsstrahlengang befindet. Hier kann die Untersuchung des Auges 7 in bekannter Art und Weise mit dem Mikroskop 5 erfolgen. D. h. der Spiegel 34 befindet sich unmittelbar vor dem Mikroskop 5, wobei die jeweiligen Strahlen für die stereoskopische Untersuchung jeweils auf beiden Seiten des schmalen Spiegels 34 vorbeiführen.

Um den breiten Spiegel 30 durch einen schmalen Spiegel 34 zu ersetzen (bzw. umgekehrt), könnten die Beleuchtungsmittel 10 auch modular ausgebildet sein. So könnte beispielsweise das Gestänge 32 derart ausgebildet sein, dass der breite Spiegel 30 alleine oder zusammen mit dem Gestänge 32 ausgewechselt werden kann. Umfasst die Umlenkvorrichtung nicht einen Spiegel, sondern ein Prisma, kann natürlich auch einfach ein anderes, schmaleres Prisma auf die Bildaufnahmevorrichtung aufgesteckt werden.

Selbstverständlich kann auch die Bildaufnahmevorrichtung 14 modular ausgebildet sein, sodass je nach Anwendung andere Bildaufnahmevorrichtungen 14, beispielsweise in Form unterschiedlicher Bildadapter für analoge oder digitale Bilder oder für Fotografien oder Videosequenzen, eingesetzt werden können. Es könnte beispielsweise auch ein Bildadapter für die Erzeugung stereoskopischer Bilder eingesetzt werden, welcher z. B. unterschiedliche Anteile des gesamten Betrachtungsstrahles, d. h. unterschiedliche oder auch teilweise überlappende Bereiche des CCD-Chips 25 verwendet, um Stereobilder des Augenfundus zu erzeugen, welche dann auf einem entsprechend ausgestatteten Monitor stereoskopisch betrachtet werden könnten.

Zusammenfassend ist festzustellen, dass es die Erfindung erlaubt, mit einem einzigen Spaltlampengerät vordere wie auch hintere Augenabschnitte in bisher nicht erreichter Qualität zu untersuchen und entsprechende Bilder auf einem Monitor darzustellen. Hierbei sind keinerlei Abstriche beim Bedienungskomfort zu machen. Soll beispielsweise von der Vordergrunduntersuchung auf die Hintergrunduntersuchung umgestellt werden, kann mit wenigen Handgriffen die Umlenkvorrichtung direkt vor dem Auge positioniert, die gewünschte Beleuchtung gewählt und der schmale durch einen breiten Spiegel ersetzt werden.

## Patentansprüche

1. Vorrichtung zur Betrachtung eines Auges (7) eines Patienten, insbesondere ein Spaltlampengerät, umfassend Betrachtungsmittel (5) zur Betrachtung eines vorderen Augenabschnittes des Auges sowie Beleuchtungsmittel zur Beleuchtung des vorderen Augenabschnittes des Auges, **dadurch gekennzeichnet, dass** die Beleuchtungsmittel derart ausgebildet sind, dass auch ein hinterer Augenabschnitt des Auges beleuchtbar ist und dass die Vorrichtung optische Abbildungsmittel zur Abbildung des hinteren Augenabschnittes sowie eine Bilderfassungsvorrichtung zur Erfassung von Bildern des Auges mit einer Bildaufnahmevorrichtung umfasst, wobei die Bildaufnahmevorrichtung derart ausgebildet und positionierbar ist, dass damit der hintere Augenabschnitt reflexfrei ophthalmoskopisch abbildbar ist.

2. Vorrichtung nach Anspruch 1, **dadurch gekennzeichnet, dass** die Beleuchtungsmittel eine Lichtquelle zur Erzeugung eines Beleuchtungsstrahles umfassen und von einer Hintergrundbeleuchtung auf eine Vordergrundbleuchtung umschaltbar sind.

3. Vorrichtung nach Anspruch 1 oder 2, **dadurch gekennzeichnet, dass** die Bilderfassungsvorrichtung eine Umlenkvorrichtung umfasst, wobei die Umlenkvorrichtung derart vor dem Auge positionierbar ist, dass ein aus dem Auge austretender Betrachtungsstrahl zur Bildaufnahmevorrichtung umlenkbar ist.

4. Vorrichtung nach einem der Ansprüche 1 bis 3, **dadurch gekennzeichnet, dass** die Beleuchtungsmittel eine Lichtquelle umfassen und mit der Bilderfassungsvorrichtung eine Einheit bilden, wobei ein von der Lichtquelle erzeugter Beleuchtungsstrahl von der Umlenkvorrichtung auf das Auge umlenkbar ist.

5. Vorrichtung nach Anspruch 4, **dadurch gekennzeichnet, dass** der Beleuchtungsstrahl unter einem veränderbaren Einfallswinkel auf das Auge umlenkbar ist.

6. Vorrichtung nach Anspruch 4 oder 5, **dadurch gekennzeichnet, dass** der Beleuchtungsstrahl eine Mehrzahl von ringförmig angeordneten Einzelstrahlen umfasst.

7. Vorrichtung nach einem der Ansprüche 1 bis 6, **dadurch gekennzeichnet, dass** die Bilderfassungsvorrichtung eine Halterung mit einer Basis und einem Träger umfasst, wobei die Bildaufnahmevorrichtung auf dem Träger befestigt ist, die Basis beweglich mit der Vorrichtung verbunden ist und die Halterung Führungsmittel aufweist zur Verschiebung des Trägers relativ zur Basis.

8. Vorrichtung nach Anspruch 7, **dadurch gekennzeichnet, dass** die Führungsmittel schienenartig ausgebildet sind und der Träger mit der Bildaufnahmevorrichtung translatorisch entlang der Basis in Richtung des Auges verschiebbar ist.

9. Vorrichtung nach einem der Ansprüche 1 bis 8, **dadurch gekennzeichnet, dass** die Bilderfassungsvorrichtung eine Bilddarstellungsvorrichtung umfasst zur Darstellung der von der Bilderfassungsvorrichtung erfassten Bilder.

10. Vorrichtung nach Anspruch 9, **dadurch gekennzeichnet, dass** die Bilddarstellungsvorrichtung eine Foto-, eine Video- oder eine CCD-Kamera umfasst.

11. Vorrichtung nach einem der Ansprüche 1 bis 10, **dadurch gekennzeichnet, dass** die optischen Abbildungsmittel zur Abbildung des hinteren Augenabschnittes augennah positioniert sind.

12. Vorrichtung nach einem der Ansprüche 1 bis 11, **dadurch gekennzeichnet, dass** die Bilderfassungsvorrichtung derart ausgebildet ist, dass sie zwischen wenigstens zwei Vergrösserungen umschaltbar ist.
